# EUROPEAN PATENT SPECIFICATION

(11) **EP 4 028 059 B1**
(45) Date of publication and mention of the grant of the patent: **24.12.2025**
(21) Application number: 19945356.4
(22) Date of filing: 09.09.2019
(51) Int. Cl.: A61K 47/06, A61K 31/05, A61K 47/14, A61K 47/22, A61K 47/44, C07C 13/32, C07C 39/23, C07D 311/04, A61K 31/355, A61K 9/08

(54) **STABLE MEDICINAL CANNABIDIOL COMPOSITIONS**
STABILE MEDIZINISCHE CANNABIDIOLZUSAMMENSETZUNGEN
COMPOSITIONS MÉDICINALES STABLES DE CANNABIDIOL

(43) Date of publication of application: 20.07.2022
(73) Proprietor: Cardiol Therapeutics Inc., Oakville, Ontario L6H 0G5 (CA)
(72) Inventor: RISTEVSKI, Blagoja, Oakville, Ontario L6M 3P1 (CA); BOLTON, Anthony Ernest, Oakville, Ontario, L6H 0G5 Canada (CA)
(74) Representative: ABG Intellectual Property Law, S.L.
(86) International application number: PCT/CA2019/051259
(87) International publication number: WO 2021/046628

(56) References cited:
- WO-A1-2017/149387
- WO-A1-2018/222923
- WO-A1-2019/089583
- CA-A1- 2 950 424
- CA-A1- 2 982 250
- US-A1- 2017 266 153
- A.-L. KLAUKE ET AL: "The cannabinoid CB2 receptor-selective phytocannabinoid beta-caryophyllene exerts analgesic effects in mouse models of inflammatory and neuropathic pain", EUROPEAN NEUROPSYCHOPHARMACOLOGY, vol. 24, no. 4, 1 April 2014 (2014-04-01), NL, pages 608 - 620, XP055600433, ISSN: 0924-977X, DOI: 10.1016/j.euroneuro.2013.10.008

## Description

### FIELD

The present specification relates to oral cannabinoid compositions for use as medicines.

### BACKGROUND

Many studies have demonstrated the therapeutic potential of cannabinoid therapies. As a result, cannabinoids have been used or explored for use in treating various diseases, conditions, disorders and/or their symptoms, including chronic pain, neuropathic pain, epilepsy and the like (e.g. Dravet Syndrome, Lennox Gastaut Syndrome, mycolonic seizures, juvenile mycolonic epilepsy, refractory epilepsy, juvenile spasms, West syndrome, refractory infantile spasms, infantile spasms), tuberous sclerosis complex, brain tumors (e.g. Glioblastoma multiforme, or GBM), cannabis use disorder, post-traumatic stress disorder, anxiety, early psychosis, schizophrenia, Alzheimer's Disease, autism, and withdrawal from opioids, cocaine, heroin, amphetamines, and nicotine.

There are at least 113 different cannabinoids isolated from the *Cannabis sativa* plant, exhibiting varied effects. These include tetrahydrocannabinol (THC), cannabidiol (CBD) and cannabinol (CBN). THC is the principal psychoactive constituent, whereas cannabidiol (CBD) does not produce psychotropic effects. As such, the use of CBD for medicinal purposes can be advantageous.

For ease of administration, it is preferable, in some cases, to administer CBD orally. However, challenges with oral based therapies include variable potency; instability of the cannabinoid in the formulation; unpredictability of response by the oral route; and imprecise dosing. These problems are compounded by the variable quality of CBD-based products in the marketplace, particularly those that are made using plant extracts. For example, in one study by Noramco Inc. (headquartered in Delaware, USA), it was found that impurities of up to 4.39% were present in several available botanical CBD products on the marketplace. Similarly, a Journal of the American Medical Association (JAMA) study found 69% of 84 cannabidiol botanical extract products were mislabeled and contained unacceptably high amounts of THC (greater than 4000 parts per million), enough to produce intoxication or impairment. CBD can be chemically synthesized; however, existing methods can produce synthetic CBD of variable purity. Commercially available synthetic CBD compositions typically limit the THC (present as an impurity) to ≤1000, 100, or 10 ppm. However, for pharmaceutical applications requiring high and/or frequent dosing, it would be desirable to reduce the level of THC present as an impurity to as low as possible.

US 2017/266153 A1 discloses a composition comprising a purified cannabinoid and a compound selected from a second purified cannabinoid, a purified terpene, and a purified flavonoid; a particular composition comprises purified CBD and beta-caryophyllene.

To be useful and approved as a pharmaceutical product, CBD-based compositions must provide safe and consistent dosing and be therapeutically effective. The potency and effectiveness of existing commercial formulations can diminish over time due to conversion of CBD to degradation products (e.g. cannabinol (CBN), THC, quinone, CBDO, etc.) during storage. Thus, a need exists to provide a medicinal (pharmaceutical grade) CBD composition that has lower amounts of THC (present as an impurity) than heretofore exists and which is stable under normal storage conditions in order to provide safe and consistent dosing. The present invention is intended to meet this need.

### SUMMARY OF INVENTION

Accordingly, the present invention provides a stable cannabidiol composition for use in oral delivery as defined in claim 1 and method for stabilizing CBD in oral compositions as defined in claim 7. The composition is substantially free of other cannabinoids, terpenes, solvents, and pharmaceutically active ingredients.

In some embodiments, the CBD can be present in an amount from about 1, 2, 3, 4, 5, 6, 7, 8, or 9 % w/w and up to about 35, 34, 33, 32, 31, 30, 29, 28, 27, 26, 25, 24, 23, 22, 21, 20, 19, 18, 17, 16, 15, 14, 13, or 12 % w/w.

In the claimed invention, the lipophilic solvent (other than the BCP) consists of a mixture of C8 and C10 triglycerides. The weight ratio of the C8 triglyceride to the C10 triglyceride can be from about 35:65 to about 85:15, about 45:55 to about 75:25, or from about 55:45 to about 65:35.

The at least one antioxidant (other than the BCP which also functions as an antioxidant) is alpha tocopherol (vitamin E). Alpha tocopherol (Vitamin E) is present in a concentration of from about 0.5, or 0.7 and up to about 1.5, 1.3, or 1.1 % w/w. In some embodiments, the alpha tocopherol is present in an amount from about 0.75 to about 1.25 % w/w.

In some embodiments, THC can be present in an amount not exceeding 0.5, 0.4, 0.3, 0.2, or 0.1 % w/w based on the weight of the CBD, and/or not more than 10, 9, 8, 7, 6, 5, 4, 3, 2, or 1 ppm based on the total composition.

In a particularly preferred embodiment, the composition consists of:
a. synthetic CBD having a purity of at least 99.8% w/w based on the weight of the CBD, in a concentration of from about 2 % w/w to about 12 % w/w;
b. BCP in a concentration of from about 28 % w/w to about 30 % w/w;
c. a mixture of C8 and C10 triglycerides in an amount from about 56 % w/w to about 60 % w/w, wherein the weight ratio of the C8 triglyceride to the C10 triglyceride is from about 55:45 to about 65:35;
d. alpha tocopherol (Vitamin E) in a concentration of from about 0.5 % w/w to about 1.5 % w/w; and
e. (optionally) from 0 % w/w to an effective amount of at least one pharmaceutically acceptable excipient;
wherein THC is absent or present as an impurity in an amount less than 1 ppm based on the total composition.

The present compositions and compositions formed by the present methods are substantially free of compounds selected from the group consisting of 3-carene, carene, humulene, α-pinene, β-pinene, linalool, limonene, γ-terpinene, terpinene, terpineol, borneol, eucalyptol, pulegone, sabinene, ocimene, camphene, bisabolol, α-bisabolol, caryophyllene oxide, terpinolene, phytol, nerolidol, myrcene, isophytol, citronellol, fenchol, geraniol, guaiol, isopulegol, menthol, p-cymene, phyllandrene, valencene, tetrahydrocannabinol (THC), cannabidolic acid (CBDA), cannabigerolic acid (CBGA), cannabinol (CBN), cannabinolic acid (CBNA), cannabigerol (CBG), cannabichromene (CBC), cannabichromenic acid (CBCA), cannabicyclol (CBL), cannabicyclolic acid (CBLA), cannabivarin (CBV), tetrahydrocannabivarin (THCV), cannabidivarin (CBDV), cannabichromevarin (CBCV), cannabigerovarin (CBGV), Nabilone, Dronabinol, tetrahydrocannabinolic acid (THCA), cannabigerol monomethyl ether (CBGM), cannabielsoin (CBE), cannabicitran (CBT), and cannabidiol-dimethylheptyl (CBD-DMH), N-[8-(2-hydroxybenzoyl) amino] caprylate (SNAC)], additional solvents, and additional pharmaceutically active agents.

These and other aspects of the present specification will be described more fully below.

### DETAILED DESCRIPTION

### Definitions

For the sake of clarity and to avoid ambiguity, certain terms are defined herein as follows.

The term "pharmaceutically active agent" means any composition of matter (e.g. agent, compound, or ingredient) capable of providing a therapeutic effect (e.g. healing, alleviating, preventing a disease or its symptoms) to a subject, including drugs, cells, DNA, RNA, oligonucleotides, proteins, and peptides.

When a composition of matter, e.g. a compound or ingredient, is described as having a "purity of X %" this means that one or more impurities may be present in an amount up to 100-X % by weight, based on the total weight of the composition of matter. The purity of an ingredient can be determined by high performance liquid chromatography (HPLC), e.g. by the area normalization of an HPLC or GC-FID profile.

The term "subject" means members of the animal kingdom including humans and other mammals.

When used herein, the term "treatment" and "prevention" are intended to mean "improving quality of life" or "extending the life" of a subject and not necessarily "curing" a condition, disorder or disease.

"Pharmaceutically acceptable excipient" when used herein means any substance which can be formulated or that is present alongside a pharmaceutically active agent to achieve a desired function or functions. Examples include colouring agents, flavouring agents, and preservatives. To be "pharmaceutically acceptable", the excipient must be non-toxic, safe for human and animal consumption, and compatible with the other ingredients in the composition, having regard to the oral mode of administration. The person skilled in the art will appreciate what compounds or ingredients would qualify as a pharmaceutically acceptable excipient given the teaches of the present specification and information in the public domain.

As used herein, the terms "stability," "stable" or the like, when used in association with compositions according to the present invention, mean that the CBD in the composition remains unchanged, i.e. undegraded, for at least 8 weeks when stored in a sealed container, away from light, at 40°C and at a relative humidity of 75%. Under refrigerator conditions of 5°C±3°C, such compositions can be expected to be stable for at least eight months, assuming all other conditions are the same. Compositions according to the present invention remain as a clear uniform liquid that will allow consistency of dosing, as shown in the below examples.

The phrases "at least one," "one or more," and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together.

The terms "a" or "an" entity refers to one or more of that entity. As such, the terms "a" (or "an"), "one or more" and "at least one" can be used interchangeably herein. It should also be noted that the term "or" is generally employed in the sense of "and/or" unless the context clearly dictates otherwise.

The term "comprising" means "including without limitation." Thus, a composition comprising a list of ingredients may include additional ingredients not expressly recited. It is also to be noted that the terms "comprising," "including," and "having" can be used interchangeably.

The term "consisting of" means "including the listed ingredients and such additional ingredients as may be present in the listed ingredients as natural or commercial impurities or additives." Natural and commercial impurities and additives will be apparent to the person of ordinary skill in the art. As noted above, synthetic cannabidiol (CBD) may contain up to about 0.5 % w/w of impurities such as residual solvents and by-products of the manufacturing process. Therefore, a composition "consisting of synthetic CBD" means that the composition has at least about 99.5 % w/w of CBD and up to about 0.5 wt.% impurities. The term "consisting essentially of" means "including the listed ingredients and any additional ingredients that do not materially affect the basic and novel properties." By "basic and novel' properties" is meant the stability of the CBD in the composition and the presence of THC in an amount less than 10 ppm based on the total composition.

Unless stated otherwise, the term "weight percent," "% w/w," "percent by weight," "% by weight," % w/w, and variations thereof, refer to the amount of a substance as the weight of that substance divided by the total weight of the composition containing that substance, and multiplied by 100.

Unless stated otherwise, the term "volume percent," "vol. %," "percent by volume" "% by volume" % v/v, and variations thereof, refer to the amount of a substance as the volume of that substance divided by the total volume of the composition containing that substance, and multiplied by 100.

The term "about" refers to variations in an expressed numerical quantity that can occur, for example, through measuring and liquid handling procedures used for making pharmaceutical compositions, differences in the manufacture, source, or purity of the ingredients used to make the compositions, and/or differences due to different equilibrium conditions or different reaction levels for a composition resulting from an initial mixture. For the sake of clarity, the term "about" includes variations in the expressed value up to ±5%. Whether or not a value is modified by the term "about," the claims include equivalents to the values.

When used herein, the term "effective amount" means an amount that would bring about the desired effect, based on the known purpose and function of the ingredient, and application of the composition. What constitutes an effective amount will be determinable by the person of ordinary skill in the art without having to engage in inventive experimentation.

The values recited herein are intended to include all values that meet the stated parameters including those not expressly recited. Thus, for example, a value of less than 1.0 % w/w is intended to include less than 0.99 % w/w, less than 0.98 wt.%, less than 0.97 wt.%, less than 0.90 % w/w, less than 0.84 % w/w, less than 0.56 % w/w, less than 0.01 % w/w, etc. Thus, all ranges disclosed herein are to be understood to encompass any and all subranges subsumed therein. For example, a stated range of "1 to 10" should be considered to include any and all subranges between (and inclusive of) the minimum value of 1 and the maximum value of 10, e.g., 1 to 6.3, or 5.5 to 10, or 2.7 to 6.1, etc.

The present specification contemplates the possibility of omitting any components listed herein. The present specification further contemplates the omission of any components even though they are not expressly named as included or excluded from the specification.

The chemical structures herein are drawn according to the conventional standards known in the art. Thus, where an atom, such as a carbon atom, as drawn appears to have an unsatisfied valency, then that valency is assumed to be satisfied by a hydrogen atom, even though that hydrogen atom is not necessarily explicitly drawn. The structures of some of the compounds of this specification include stereogenic carbon atoms. It is to be understood that isomers arising from such asymmetry (e.g., all enantiomers and diastereomers) are included within the scope of this specification unless indicated otherwise. That is, unless otherwise stipulated, any chiral carbon center may be of either (R)- or (S)-stereochemistry. Such isomers can be obtained in substantially pure form by classical separation techniques and by stereochemically-controlled synthesis. Furthermore, alkenes can include either the E- or Z-geometry, where appropriate.

### CANNABINOID COMPOSITION

The present compositions are free of botanical drug substances. A "botanical drug substance" or "BDS" is defined herein to mean a drug derived from one or more plants, algae, or microscopic fungi by processes such as pulverization, decoction, expression, aqueous extraction, ethanolic extraction and the like. However, excluded from the definition of BDS are biosynthetic CBD, i.e. CBD derived from genetically modified yeasts, bacteria, and the like and grown in culture. The cannabidiol used in the present compositions are produced by chemical synthesis with a high level of purity to enhance safety and consistency of dosing.

### Synthetic Cannabidiol (CBD)

The terms cannabidiol and CBD are used interchangeably herein and unless the context dictates otherwise refer to synthetic cannabidiol.

"Synthetic cannabinoids" are compounds that have a cannabinoid-like structure yet are manufactured using chemical means. The synthetic cannabidiol used in the present cannabinoid compositions possess the chemical structure shown below:

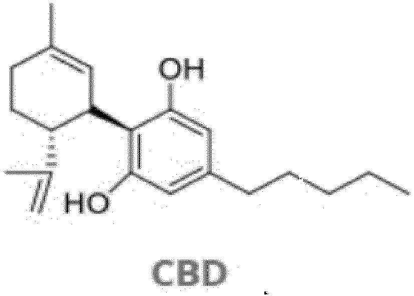

Methods of manufacturing synthetic cannabidiol are known in the art. For example, the CBD from NORAMCO, INC. headquartered in Wilmington Delaware, U.S.A. is made according to processes such as those described in U.S. patent publication US 2017/0008868 A1 (granted as U.S. patent 10,059,683) and US 2018/031,976 A1, incorporated herein by reference. Synthetic CBD made by other processes and manufacturers can also be used to make the present compositions provided they have a purity of at least 99.5, 99.6, 99.7, 99.8, or 99.9 %.

The terms tetrahydrocannabinol, THC, delta-9-tetrahydrocannabinol, and delta-9-THC are used interchangeably herein and refer to the chemical compound having the structure shown hereinbelow. The term is used broadly herein to include the double bond isomers and their stereoisomers.

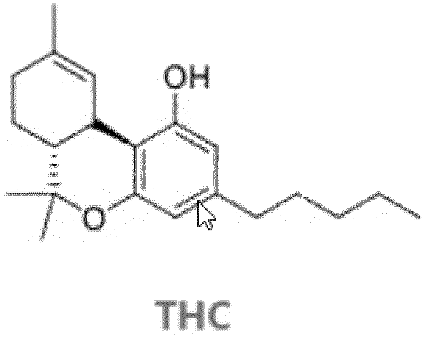

The skilled person will appreciate that commercial sources of synthetic CBD will contain minor amounts of impurities such as residual solvents and by-products of manufacture, e.g. olivetol, monobromo-CBD, and delta-9-THC. Residual solvents include methanol, n-heptane, dichloromethane, and triethylamine.

Compositions according to the invention are "substantially free" of THC. When used herein, a composition that is "substantially free" of "Y" means that "Y" is either not present or is present in an amount less than 0.5 % w/w based on the total composition. Embodiments of CBD compositions according to the invention either do not contain THC, or contain THC in an amount less than 10, 9, 8, 7, 6, 5, 4, 3, 2, or 1 ppm.

### Carrier - BCP and at Least One Additional Lipophilic Compound

β-caryophyllene (BCP), also named trans-(1R,9S)-8-Methylene-4,11,11-trimethylbicyclo[7.2.0]undec-4-ene or [1R-(1R,4E,9S)]-4,11,11-trimethyl-8-methylene-bicyclo[7.2.0]undec-4-ene, is a natural bicyclic sesquiterpene compound found in botanical extracts of plants, including *Cannabis sativa.* It is a constituent of many essential oils, especially clove *Syzygium aromaticum* oil and is "generally recognized as safe" (GRAS).

Caryophyllene is the only terpene known to interact with the endocannabinoid system (at CB2 receptors). β-caryophyllene selectively binds to the CB2 receptor and is a functional CB2 agonist. Furthermore, β-caryophyllene has been identified as a functional non-psychoactive CB2 receptor ligand in foodstuff and as a macrocyclic anti-inflammatory cannabinoid in cannabis. (Proc Natl Acad Sci USA. 2008 Jul. 1; 105(26):9099-104. doi: 10.1073/pnas.0803601105. Epub 2008 Jun. 23. Beta-caryophyllene is a dietary cannabinoid.). BCP is used as a flavouring agent, antioxidant, penetration enhancer via gastrointestinal mucosa, anti-inflammatory agent, and solvent (see, e.g., WO2002034294 to Schwankl et al). It may be useful in improving the systemic availability of CBD (WO2002034275A1 to Schwankl et al.). It is predicted that compositions according to the present invention will provide enhanced absorption of CBD into the subject's bloodstream and therefore enhanced therapeutic efficacy. This is based on the theory that BCP can block or inhibit enzymes released by bacteria in the gut that expedite degradation of CBD. By impeding CBD degradation in the gastrointestinal tract, a greater percentage of the administered dose of CBD should become bioavailable.

Surprisingly, the inventors have found that BCP is a highly effective solvent of CBD and can also enhance the stability of CBD when used in combination with at least one additional lipophilic solvent in the amounts described herein. For example, at least 300 mg of CBD can be dissolved in 1 mL of BCP and its derivatives. The enhanced stability of the CBD provides a composition with an acceptable shelf life and which can provide consistency in dosing and enhanced safety.

Without being bound by theory, it is believed that the presence of BCP in the amounts disclosed herein impedes degradation of the at least one additional lipophilic compound (e.g. MCT) into free fatty acids which can interact with moisture (either present as an impurity in the ingredients used to make the composition or in the atmosphere) to produce an acidic environment that can cause CBD to convert to THC. The BCP in the present compositions appears to act synergistically with Vitamin E to impede this conversion thereby providing a more stable CBD product that is substantially free of THC. In preferred embodiments, THC is not present or is present in an amount less than 10, 9, 8, 7, 6, 5, 4, 3, 2, or 1 ppm based on the total composition.

The relative amounts of BCP and its derivatives to the cannabidiol can vary. For example, for every 1 mL of BCP and derivatives, the amount of the cannabidiol can vary from about 1 mg, 2 mg, 5 mg, or 10 mg, and up to about 300 mg, 275 mg, 200 mg, or 100 mg. The weight ratio of CBD to BCP and its derivatives can be about 1:5, 1:4, 1:3, 1:2, or 1: 1. The volume ratio of BCP to the at least one additional lipophilic solvent as defined in claim 1 is from about 1:1 to about 1:3, preferably about 1:2. BCP and the at least one additional lipophilic solvent together form a carrier for the CBD. Lipophilic solvents that can be used with BCP must be acceptable for human consumption.

The composition contains a blend of BCP and certain medium chain triglycerides (MCTs). The medium chain triglyceride may be synthetic or natural (e.g., produced from fractionated oils, such as coconut oil and/or palm kernel oil). "Medium chain triglyceride" refers to esters of glycerol having three C6 to C12 fatty acid chains, where the three fatty acid chains may be the same or different. Medium chain triglycerides are represented by the following formula:

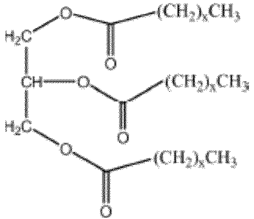

wherein each x is independently 4, 6, 8, or 10. When x is 4, the chain is referred to as a C6 fatty acid. When x is 6, the chain is referred to as a C8 fatty acid. When x is 8, the chain is referred to as a C10 fatty acid. When x is 10, the chain is referred to as a C12 fatty acid. In various embodiments, each x is the same integer; two x are the same integer and one x is a different integer; or each x is a different integer.

In various embodiment, the medium chain triglyceride comprises esters of (i) three C8 fatty acids; (ii) three C10 fatty acids; (iii) two C8 fatty acids and one C10 fatty acid; (iv) two C10 fatty acids and one C8 fatty acid;. In one embodiment, the medium chain triglyceride comprises two C8 fatty acids and one C10 fatty acid. In one embodiment, the medium chain triglyceride comprises two C10 fatty acids and one C8 fatty acid.

The skilled artisan will appreciate that a mixture of medium chain triglycerides may result from any process (e.g., fractionation, hydrogenation) used to prepare medium chain triglycerides. For example, substantially all the medium chain triglycerides obtained from fractionated coconut oil may comprise C8 and/or C10 fatty acids; however, there may be some medium chain triglycerides (not encompassed by the invention) containing C6 and/or C12 fatty acids.

The carrier may comprise one, two, three, four or more different medium chain triglycerides. In one embodiment, the carrier comprises a medium chain triglyceride comprising esters of two C8 fatty acids and one C10 fatty acid. In one embodiment, the carrier comprises a medium chain triglyceride comprising esters of one C8 fatty acid and two C10 fatty acids. In one embodiment, the carrier comprises two different medium chain triglycerides, where a first medium chain triglyceride comprises esters of two C8 fatty acids and one C10 fatty acid and a second medium chain triglyceride comprises esters of one C8 fatty acid and two C10 fatty acids.

The triglycerides may be prepared by methods known in the art and are commercially available
In a non-claimed embodiment the carrier is a propylene glycol diester of saturated vegetable fatty acids with chain lengths of C8 and C10 (caprylic and capric acid). An example of one such commercially available carrier is MIGLYOL ^{®} 840 (Sasol Germany GmbH, Witten, Germany).

Preferably, the composition comprises MCTs containing a mixture of C8 and C10 triglycerides in a ratio (C8:C10) of from about 55:45 to about 65:35, such as those available from Vigon International, Inc.

Embodiments of the invention described below use BCP from Sigma Aldrich. The product specification is included in ANNEX A (CAS Number 87-44-5). Such product contains at least 95% w/w major and minor C15H24 terpenes and up to 5% w/w impurities such as by-products of manufacturing. Thus, the BCP from Sigma Aldrich has a "purity" of at least 95%. Other embodiments of the invention can use other commercial sources of BCP having higher degrees of purity, e.g. BCP that is at least 96, 97, 98, 99, and 99.5 % pure.

The BCP and derivatives thereof used in the present composition can be of synthetic or natural origin. Preferably, synthetic BCP products are used.

When used herein, a BCP "derivative" means a C15H24 minor terpene hydrocarbon that results from the chemical synthesis of BCP according to known processes and include the minor terpenes present in the Sigma-Aldrich product.

### Antioxidants

Vitamin E refers to a group of eight water-insoluble compounds that include tocopherols and tocotrienols. When used herein, vitamin E means the following RRR alpha-tocopherol form of vitamin E:

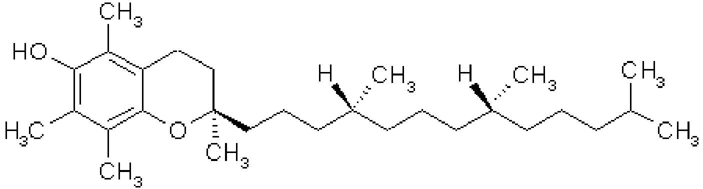

Also, vitamin E and alpha tocopherol are herein used interchangeably. Vitamin E is used as an antioxidant. The term "antioxidant" is used herein to describe any compound or combination of compounds that prevents or slows down cannabinoid oxidation. Other antioxidants not encompassed by the invention include polyphenols (phenolic acids, flavonoids, anthocyanins, lignans and stilbenes), carotenoids (xanthophylls and carotenes), propyl gallate, lecithin, curcumin, sesamin, sesamol, sesamolin, ascorbyl palmitate, butylated hydroxyanisole (BHA), and butylated hydroxytoluene (BHT). The antioxidant, e.g. vitamin E, can be used in an amount of from about 0.5, 0.6, or 0.7 % w/w and up to about 1.5, 1.25, or 1 % w/w.

The preparation may also contain and effective amount of antioxidant synergists, as are known in the art.

### Additional Pharmaceutically Acceptable Excipients

The cannabinoid composition can comprise at least one additional pharmaceutically acceptable excipient. Examples of pharmaceutically acceptable excipients are diluents, carriers, solvents, viscosity modifiers, colouring agents, flavouring agents, sweeteners, taste masking agents, stabilizers, absorption enhancers, odorants, and mixtures thereof. The choice and amount of the excipient(s) additives can be readily determined by one skilled in the art and will depend on the other ingredients in the composition, the desired properties of the final composition, oral route of administration, and need for a stable final product with the desired shelf-life.

### Preservatives

The term "preservative" when used herein means those compounds that possess bactericidal and/or fungicidal properties. Other than as expressly recited herein, the composition is substantially free of added preservatives such as parabens (e.g. methyl paraben and propyl paraben).

### Sweeteners and Flavouring Agents

The present oral compositions may further include an effective amount of a lipid soluble sweetener in an amount from about 0.001 % w/w to about 5 % w/w, about 0.01 % w/w to about 1 % w/w, or from about 0.25 % w/w to about 0.5 % w/w. The sweetener can be selected from the group consisting of saccharin, alkoxy aromatiC8, oximes, sulfamic acids, dihydrochalcones, aspartyl malonates, succanilic acids, and mixtures thereof.

Embodiments of the invention can also contain at least one lipid soluble natural or synthetic flavouring agent in an amount from about 0.1 % w/w to about 5 % w/w, about 0.1 % w/w to about 1 % w/w, or from about 0.25 % w/w to about 0.75 % w/w. The flavouring agent can be selected from the group consisting of oil of sweet birch, oil of spearmint, oil of wintergreen, anise oil, dill oil, celery seed oil, various citrus oils, including lemon, orange, lime, tangerine and grapefruit oils, clove oil, peppermint oil, cassia, carrot seed oil, cola concentrate, ginger oil, angelica oil, vanillin, and combinations thereof.

### Absorption Enhancers

Absorption enhancers may also be used such as, for example, Gelucire^{™} 44/14; Gelucire^{™} 50/13; Tagat^{™} TO; Tween^{™} 80; isopropyl myristate, polysorbates, sorbitan esters, poloxamer block copolymers, PEG-35 castor oil, PEG-40 hydrogenated castor oil, caprylocaproyl macrogol-8 glycerides, PEG-8 caprylic/capric glycerides, sodium lauryl sulfate, dioctyl sulfosuccinate, polyethylene lauryl ether, ethoxydiglycol, propylene glycol mono-di-caprylate, glycerol monocaprylate, glyceryl fatty acids (C8-C18) ethoxylated, oleic acid, linoleic acid, glyceryl caprylate/caprate, glyceryl monooleate, glyceryl monolaurate, caprylic/capric triglycerides, ethoxylated nonylphenols, PEG-(8-50) stearates, olive oil PEG-6 esters, triolein PEG-6 esters, lecithin, d-alpha tocopheryl polyethylene glycol 1000 succinate, polycarbonate, sodium glycocholate, sodium taurocholate, cyclodextrins, citric acid, sodium citrate, triacetin, combinations thereof, and the like. When used, the absorption enhancer may be present in an amount of from about 0.001 % w/w to about 10 % w/w, or from about 0.01 % w/w to about 5 % w/w.

### Colouring Agents

Other excipients that can be used are colouring agents such as red, black and yellow iron oxides and FD&C dyes such as FD&C Blue No. 2, FD&C Red No. 40, and the like.

It is recognized that pharmaceutical excipients may perform more than one function and are therefore characterized as having different uses depending on the application. While the use of an excipient in the context of a specific formulation may determine the function of the excipient, the inclusion of any excipient into any one or more category as set forth above is not meant to limit the function of that excipient.

### Dosage Forms

The cannabinoid composition can take a variety of forms all for administration to the gastrointestinal tract, including liquids for oral administration, drops, syrup, elixir, soft gel capsules, and liquid-filled two-piece capsules. The person skilled in the art reading the present disclosure will understand which forms the embodiments of the present compositions can take based on the present teachings.

As used herein, a soft gel capsule is an oral dosage form for administration of pharmaceuticals and nutraceuticals. The soft gel capsule comprises a soft gel shell which is a combination of gelatin or gelatin alternative, water, opacifier and a plasticizer to lend flexibility, such as glycerin and/or sorbitol. Soft gel capsules also will comprise a "pre-gel concentrate" which is used herein to refer to a composition that contains in a volume to be encapsulated, a single dosage or fractional dosage of CBD. As used herein, a fractional dosage refers to an amount that is less than a full dosage so that, when provided as a capsule, a plurality of capsules will be required to provide a single dosage. Typically, a fractional dosage is at least 20%, 25%, 50% of a full dosage.

### Routes of Administration

The present cannabinoid compositions are administered orally. However, other methods of administering the compositions to the gastrointestinal tract, e.g. nasogastrically, are within the scope of the present invention.

The invention may be better understood with reference to the examples below.

### EXAMPLES

The following ingredients were used to prepare solutions according to the present invention.

| **Ingredient** | **Function in formulation** | **Supplier / Catalog Number** |
|---|---|---|
| (-)-cannabidiol | Active Pharmaceutical Ingredient (API) | Noramco; 75407; CAS #13956-29-1 |
| (-)-cannabidiol | Active Pharmaceutical Ingredient (API) | BioVectra; Catalog Number 7082; Lot Number 49395; CAS #13956-29-1 |
| Beta-caryophyllene (BCP) ≥80%, FC | Co-solvent | Sigma Aldrich; W225207; CAS # 87-44-5 |
| Coconut Oil | Principal Solvent | Spectrum Chem.: C0110; CAS # 8001-31-8 |
| MCT (C8, C10 triglycerides) | Principal Solvent | Vigon International, Inc. of Stroudsberg, Pennsylvania under product code 507177 |
| Vitamin E (alpha-tocopherol, FCC) ≥95.5% | Antioxidant | Spectrum Chemicals; CAS # 10191-41-0 |

ANNEX A contains the product specification sheets and/or certificates of analysis for several of the above ingredients. The CBD from Noramco was synthesized chemically, in a crystalline powdered form, and at least 99.8% pure.

Solutions were prepared in a main formulation vessel to which was added a measured amount of the at least one additional lipophilic solvent (either coconut oil warmed to 25°C to 30°C (not encompassed by the invention), or MCT at room temperature). The solvent was stirred using a moderate vortex. To this solution was added a measured amount of BCP and the resultant mixture was stirred for 5 to 15 minutes until a homogenous solvent mixture was formed. 15% of this solvent mixture was removed for use in rinsing containers of CBD and Vitamin E in a later step. The vortex speed in the main formulation vessel was increased and then a measured amount of crystalline synthetic CBD (in powder form) was added gradually. The vessel containing the CBD was rinsed three times with part of the earlier retained solvent and the rinse was added to the main formulation vessel. The main mixture was stirred for a further 30 minutes to four hours at a higher speed until the CBD dissolved completely. During stirring of the CBD mixture, the stirrer was stopped periodically in order to check for undissolved CBD particles. After formation of a clear homogeneous CBD solution, the antioxidant (Vitamin E as a thick oily liquid) was added to the main formulation vessel. The balance of the earlier retained solvent was used to rinse the antioxidant vessel the rinse was dispensed into the main vessel. The mixture was continued to be stirred at a lower speed for at least another 10 minutes until a clear and homogeneous final solution was formed.

### Solution 1 (not encompassed by the invention)

Solution 1 is summarized in Table 1 below.

**Table 1**

| INGREDIENT | AMOUNT (g) | AMOUNT (% w/w) |
|---|---|---|
| CBD | 27 | 22.67 |
| BCP | 29.9 | 25.11 |
| Coconut oil | 61.2 | 51.41 |
| Vitamin E | 0.95 | 0.81 |

The weight ratio of CBD:BCP was between 1:1 and 1:2 and the ratio of BCP:Coconut oil was between 1:2 and 1:3, or about 1:2.

Modifications can be made to Solution 1 without departing from the scope of the invention. For example, the amount of Vitamin E can be increased to about 1, 2, 3, 4 or 5 % w/w and reducing the amount of the solvent mixture (BCP and coconut oil) by a corresponding amount.

### Solution 2

Solution 2, shown in Table 2 below, is the same as Solution 1 except for the additional lipophilic solvent.

**Table 2**

| INGREDIENT | AMOUNT (g) | AMOUNT (% w/w) |
|---|---|---|
| CBD | 27 | 22.67 |
| BCP | 29.9 | 25.11 |
| MCT | 61.2 | 51.41 |
| Vitamin E | 0.95 | 0.81 |

It can be preferable to use MCT instead of coconut oil because MCT is a liquid at room temperature, whereas coconut oil must be warmed to around 26-27°C to liquefy prior to being used to make solutions according to the invention.

### Solutions 3-5 (not encompassed by the invention)

Three 500-ml batches of cannabidiol solutions (Solutions 3-5) were prepared having target concentrations of CBD of 30 mg/ml, 100 mg/ml and 250 mg/ml, respectively. In each solution, 1 part BCP, 2 parts coconut oil and 1 % v/v of Vitamin E were employed.

The materials, target concentration, and composition of the CBD formulation prototype batches, 500 ml, are summarized in Table 3 below:

**Table 3**

| | **Solution 3 (456.04 g total weight)** | | | **Solution 4 (459.78 g total weight)** | | | **Solution 5 (465.82 g total weight)** | | |
|---|---|---|---|---|---|---|---|---|---|
| **Ingredient** | **mL** | **g** | **% w/w** | **mL** | **g** | **% w/w** | **mL** | **g** | **% w/w** |
| (-)-cannabidiol | n/a | 15.015 | 3.3 | n/a | 50.055 | 10.9 | n/a | 125.128 | 26.9 |
| BCP | 158 | 143.25 | 31.4 | 147 | 133.28 | 29.0 | 122 | 110.61 | 23.7 |
| Coconut Oil | 316 | 293.02 | 64.3 | 293 | 271.69 | 59.1 | 243 | 225.33 | 48.4 |
| Vitamin E | 5 | 4.75 | 1.0 | 5 | 4.75 | 1.0 | 5 | 4.75 | 1.0 |
| Target conc. CBD (mg/mL) | 30 | | | 100 | | | 250 | | |
| Approximate Volume Ratio of BCP : Coconut oil | 1:2 | | | 1:2 | | | 1:2 | | |

The concentration of CBD in Solutions 3-5 were assayed using a chromatographic technique and the results, shown in Table 4, are deemed to be acceptable for commercial pharmaceutical purposes.

**Table 4**

| **Solution** | **Target CBD concentration (mg/mL) ["Label Claim"]** | **Measured CBD concentration (mg/mL)** | **% Label Claim** |
|---|---|---|---|
| **3** | 30.0 | 28.8 | 96.0 |
| **4** | 100.0 | 100.3 | 100.3 |
| **5** | 250.0 | 249.7 | 99.9 |

### STABILITY TESTS

Solutions 6-8 (Lots 180-2842, 181-2842, and 182-2842) according to further embodiments of the invention were prepared and summarized in Table 5 below.

**Table 5**

| | **Solution 6** | | **Solution 7** | | | **Solution 8** | |
|---|---|---|---|---|---|---|---|
| **Ingredient** | **g** | **% w/w** | **g** | **% w/w** | | **g** | **% w/w** |
| CBD | 125.302 | 2.754 | 501.0 | 10.955 | | 125.3 | 2.754 |
| BCP | 1459.2 | 32.070 | 1341.7 | 29.338 | | 1459.3 | 32.072 |
| MCT | 2918 | 64.132 | 2683 | 58.668 | | 2918 | 64.13 |
| Vit. E | 47.502 | 1.044 | 47.504 | 1.039 | | 47.50 | 1.044 |
| Batch size (L) | 5 | | 5 | | | 5 | |
| CBD target concentration (mg/mL) | 25 | | 100 | | | 25 | |

Solutions 6 and 7 used synthetic CBD from Noramco (Lot # 004803), while Solution 8 used synthetic CBD from BioVectra Inc. of Charlottetown, P.E.I. (Catalogue Number 7082, Lot Number 49395; CAS Registry Number 13956-29-1). Both the synthetic CBD from Noramco and from BioVectra were at least 99.8 % pure.

Solutions 6-8 were each divided into 40 samples. 10 samples of each formulation were stored in 1 oz. amber glass boston round 200/400 bottles using as the closure a CR cap (20 mm, PE foam liner 20/40). These samples were stored right side up (RSU). Another 10 samples of each formulation were stored in the same containers oriented upside down (USD). Still another 10 samples of each formulation were stored in polyethylene (PET) boston round 20/400 bottles using as the closure a CR cap (20 mm Pictorial White, PE foam liner 20/400), oriented right side up (RSU). Finally, another 10 samples of each formulation were stored in the same bottles, oriented upside down (USD). All samples were stored at 40°C±2°C at relative humidity (RH) 75%±5% in a chamber (DCL 004065) according to protocol number STA-2842-7119.

The amount of CBD, BCP and THC was assayed using high performance liquid chromatography (HPLC) at time = 0, 2, 4, 6, and 8 weeks. The percentage of the label claim (% LC) for the CBD and BCP was determined and the average results for all 10 bottles are summarized in Tables 6, 7, and 8. These tables also show the limit of the THC in the samples expressed in terms of ppm based on the total composition.

**Table 6 - Solution 6**

| Time | | T=0wk | T=2 wk | T=4 wk | T=6 wk | T=8 wk |
|---|---|---|---|---|---|---|
| Glass Bot RSU | CBD | 99.6 | 100 | 99.5 | 99.4 | 104.1 |
| | BCP | 98.9 | 98.6 | 98.4 | 99.0 | 99.6 |
| | Limit THC (ppm) | N/A¹ | 0.14 | 0.003 | 0.06 | 0.07 |
| Glass Bot USD | CBD | 99.6 | 100.3 | 99.9 | 98.1 | 103.7 |
| | BCP | 98.9 | 98.8 | 98.6 | 97.8 | 99.2 |
| | Limit THC (ppm) | N/A¹ | 0.27 | 0.004 | 0.05 | 0.07 |
| PET Bot RSU | CBD | 99.6 | 99.3 | 99.9 | 99.9 | 101.8 |
| | BCP | 98.9 | 97.8 | 98.5 | 99.3 | 97.8 |
| | Limit THC (ppm) | N/A¹ | 0.15 | 0.004 | 0.05 | 0.10 |
| PET Bot USD | CBD | 99.6 | 100.2 | 100.5 | 94.8 | 102.3 |
| | BCP | 98.9 | 98.7 | 98 | 93.8 | 96.6 |
| | Limit THC (ppm) | N/A¹ | 0.13 | 0.007 | 0.05 | 0.10 |

| | | | | | | |
|---|---|---|---|---|---|---|
| ¹ The THC peak was misidentified due to insufficient chromatographic separation during the T=0 testing. | | | | | | |

The mean percentage LC for CBD for Solution 6 was 100.105 ±0.390224 std. deviation (across time periods), and ±1.597686 std. deviation (across bottle types and orientations).

**TABLE 7 - Solution 7**

| Time | | T=0 wk | T=2 wk | T=4 wk | T=6 wk | T=8 wk |
|---|---|---|---|---|---|---|
| Glass Bot RSU | CBD | 99.1 | 99.2 | 92.1 | 97.6 | 101.5 |
| | BCP | 99.4 | 98.9 | 92.0 | 98.5 | 98.2 |
| | Limit THC (ppm) | N/A¹ | 0.46 | 0.038 | 0.16 | 0.18 |
| Glass Bot USD | CBD | 99.1 | 97.1 | 99 | 98.2 | 101.2 |
| | BCP | 99.4 | 96.6 | 98.8 | 99 | 97.9 |
| | Limit THC (ppm) | N/A¹ | 0.41 | 0.025 | 0.15 | 0.16 |
| PET Bot RSU | CBD | 99.1 | 99.3 | 99.1 | 95.8 | 102.7 |
| | BCP | 99.4 | 99.1 | 99 | 96.7 | 99.5 |
| | Limit THC (ppm) | N/A¹ | 0.37 | 0.024 | 0.14 | 0.14 |
| PET Bot USD | CBD | 99.1 | 99 | 98.9 | 99.4 | 102.8 |
| | BCP | 99.4 | 98.6 | 98.7 | 100.3 | 99.5 |
| | Limit THC (ppm) | N/A¹ | 0.29 | 0.053 | 0.13 | 0.14 |

| | | | | | | |
|---|---|---|---|---|---|---|
| ¹ The THC peak was misidentified due to insufficient chromatographic separation during the T=0 testing. | | | | | | |

For Solution 7, the mean percentage LC for CBD was 98.965 ±0.699482 std. deviation (across time periods), and ±1.671347 std. deviation (across bottle types and orientations).

**Table 8 - Solution 8**

| Time | | T=0 wk | T=2 wk | T=4 wk | T=6 wk | T=8 wk |
|---|---|---|---|---|---|---|
| Glass Bot RSU | CBD | 95 | 100.1 | 99.8 | 100.6 | 104.3 |
| | BCP | 103.4 | 98.6 | 98.4 | 100.4 | 99.8 |
| | Limit THC (ppm) | N/A¹ | 0.13 | 0.003 | 0.07 | 0.07 |
| Glass Bot USD | CBD | 95 | 100.3 | 99.8 | 102.6 | 104.3 |
| | BCP | 103.4 | 98 | 98.4 | 101.2 | 98.8 |
| | Limit THC (ppm) | N/A¹ | 0.07 | 0.002 | 0.06 | 0.08 |
| PET Bot RSU | CBD | 95 | 100.5 | 99.8 | 102.2 | 104.0 |
| | BCP | 103.4 | 98.8 | 98.4 | 102.3 | 99.4 |
| | Limit THC (ppm) | N/A¹ | 0.25 | 0.005 | 0.06 | 0.07 |
| PET Bot USD | CBD | 95 | 100.2 | 100.6 | 102.4 | 104.6 |
| | BCP | 103.4 | 97.9 | 97.8 | 101.6 | 99.2 |
| | Limit THC (ppm) | N/A¹ | 0.15 | 0.034 | 0.06 | 0.07 |

| | | | | | | |
|---|---|---|---|---|---|---|
| ¹ The THC peak was misidentified due to insufficient chromatographic separation during the T=0 testing. | | | | | | |

For Solution 8, the mean percentage LC for CBD was 100.305 ±0.219716 std. deviation (across time periods), and ±3.062777 std. deviation (across bottle types and orientations).

The above results showed no trend of CBD loss over time, regardless of the type of vessel and cap closure used to store the CBD. Also, THC levels for all samples did not exceed 1 ppm during the period of the test. These results suggest that Solutions 6-8 should remain stable under long term storage conditions (at 25°C ±2°C and relative humidity of 60°C away from light) for at least 16 weeks and much longer if stored in a refrigerator (at 5°C±3°C) or a freezer at (-20°C±5°C). The extremely low level of THC in the samples show that these formulations should be safe and should provide consistent dosing in medical applications.

Solutions 6-8 were also inspected visually at time = 0, 2, 4, 6, and 8 weeks. At all time points, all samples of these solutions appeared as a homogeneous, pale, yellow clear liquid and passed the visual inspection tests.

### Methods of Treating a Condition, Disorder, Disease or Symptom Thereof

The present cannabinoid compositions are intended to be used in any therapy in which cannabidiol is indicated, including, without limitation, the treatment of cancer (e.g. Glioblastoma multiforme), cardiovascular disease (e.g. heart failure with preserved ejection fraction (HFpEF)), anxiety, autism, seizures, chronic pain, psychosis, arthritis and other diseases or disorders involving inflammation.

The amount of CBD to be administered will vary by body weight and nature of the condition. The dosage regime can entail the administration of 0.1 to 30 mg of CBD / kg body weight per day, preferably 10-20 mg of CBD per kg body weight per day. The dose can be divided for administration 2, 3, or 4 times a day.

The foregoing description of embodiments is by way of example only and is not intended to limit the scope of the invention as herein described and claimed.

### ANNEX A

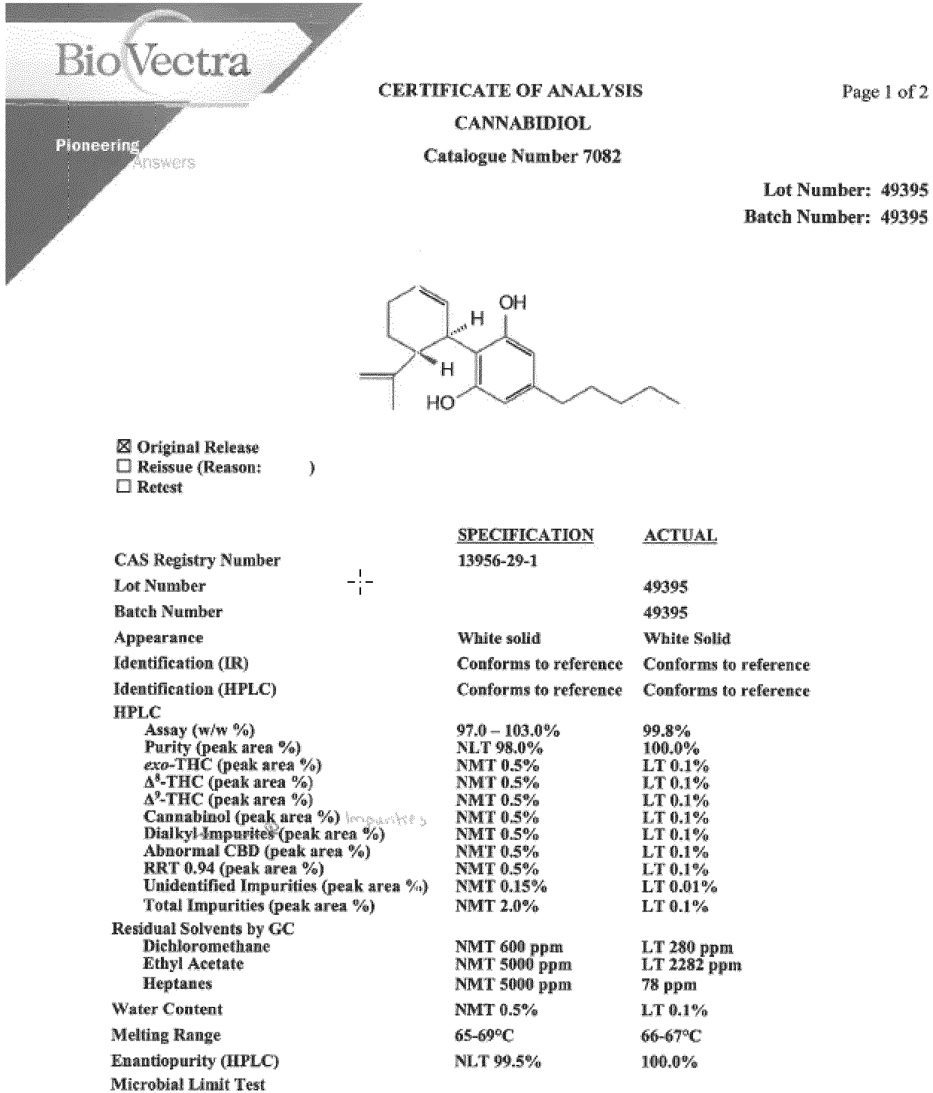

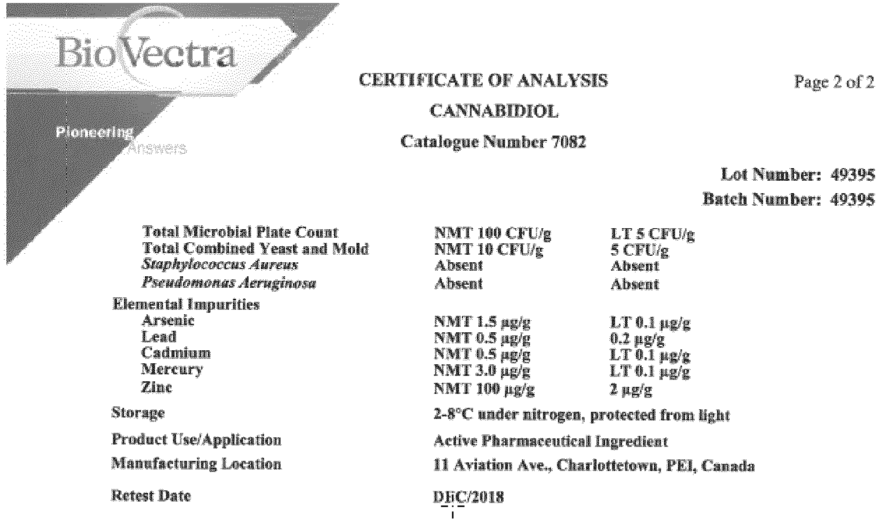

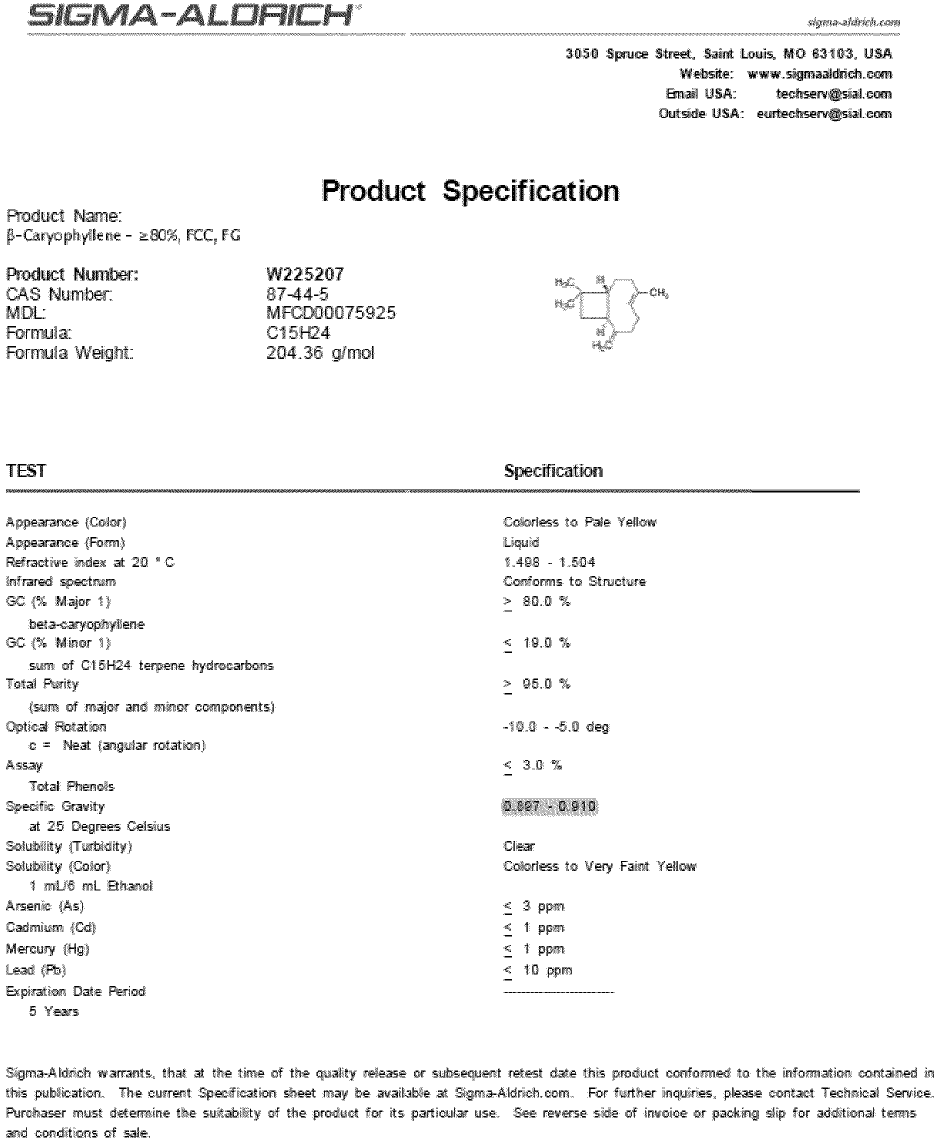

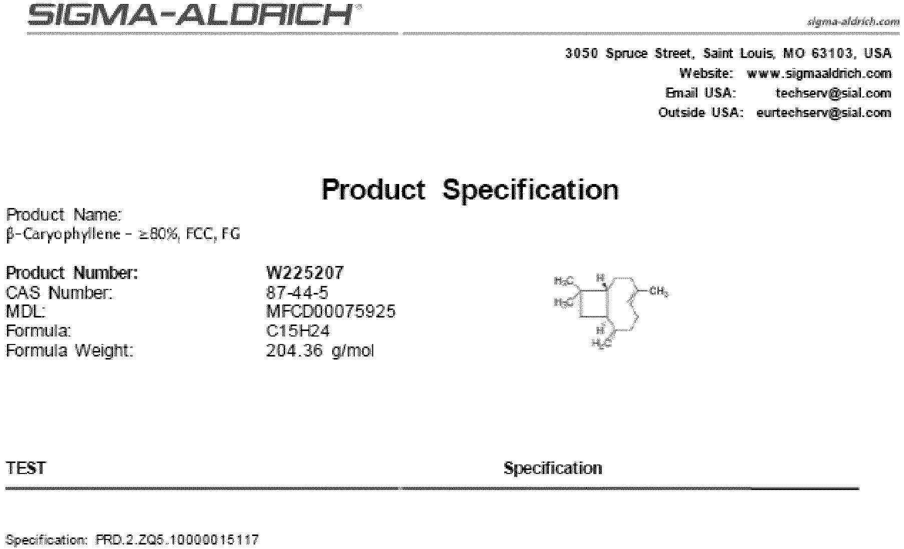

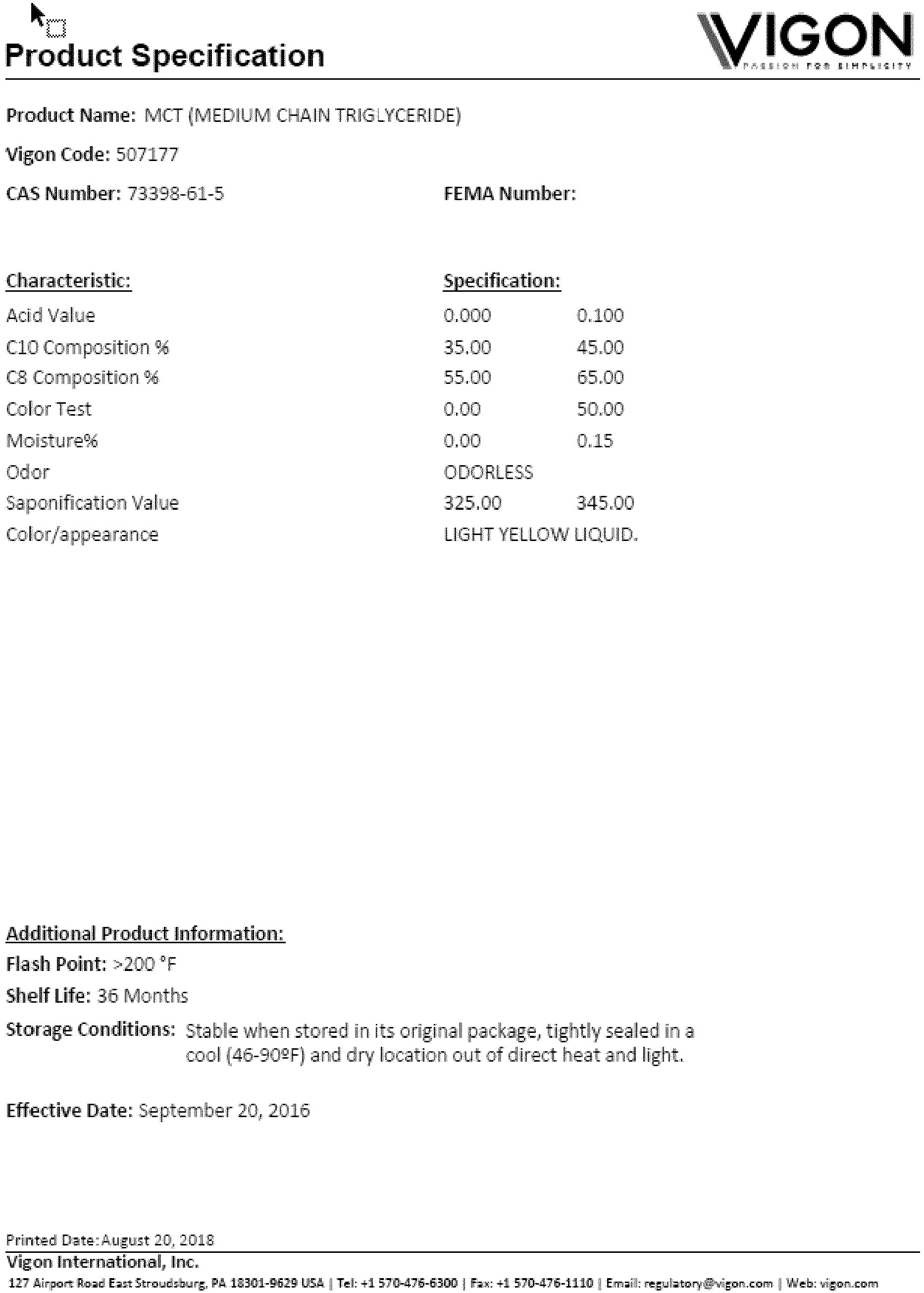

## Claims

1. A stable cannabidiol composition for use in oral delivery, the composition comprising:
a. synthetic cannabidiol (CBD) having a purity of at least 99.5 % based on the weight of the CBD, in a concentration of from about 1 % w/w to about 35 % w/w;
b. a lipophilic carrier in a concentration of from about 64 % w/w to about 98 % w/w, wherein the carrier consists of beta-caryophyllene (BCP) and a mixture of C8 and C10 triglycerides, wherein the volume ratio of BCP to the mixture of C8 and C10 triglycerides is from about 1:1 to about 1:3;
c. alpha tocopherol (vitamin E), in a concentration of from about 0.5 % w/w to about 1.5 % w/w; and
d. (optionally) from 0 % w/w to an effective amount of at least one pharmaceutically acceptable excipient;
wherein the composition is substantially free of compounds selected from the group consisting of 3-carene, carene, humulene, α-pinene, β-pinene, linalool, limonene, γ-terpinene, terpinene, terpineol, borneol, eucalyptol, pulegone, sabinene, ocimene, camphene, bisabolol, α-bisabolol, caryophyllene oxide, terpinolene, phytol, nerolidol, myrcene, isophytol, citronellol, fenchol, geraniol, guaiol, isopulegol, menthol, p-cymene, phyllandrene, valencene, tetrahydrocannabinol (THC), cannabidolic acid (CBDA), cannabigerolic acid (CBGA), cannabinol (CBN), cannabinolic acid (CBNA), cannabigerol (CBG), cannabichromene (CBC), cannabichromenic acid (CBCA), cannabicyclol (CBL), cannabicyclolic acid (CBLA), cannabivarin (CBV), tetrahydrocannabivarin (THCV), cannabidivarin (CBDV), cannabichromevarin (CBCV), cannabigerovarin (CBGV), Nabilone, Dronabinol, tetrahydrocannabinolic acid (THCA), cannabigerol monomethyl ether (CBGM), cannabielsoin (CBE), cannabicitran (CBT), and cannabidiol-dimethylheptyl (CBD-DMH), N-[8-(2-hydroxybenzoyl) amino] caprylate (SNAC)], additional solvents, and additional pharmaceutically active agents.

2. The composition of claim 1, wherein the volume ratio of BCP to the mixture of C8 and C10 triglycerides is about 1:2.

3. The composition of claim 1 or 2, wherein the CBD is present in an amount from about 2 % w/w to about 25 % w/w.

4. The composition of claim 3, wherein the CBD is present in an amount from about 9 % w/w to about 23 % w/w.

5. The composition of any one of claims 1 to 4, wherein the CBD is at least 99.8% pure.

6. The composition of any one of claims 1 to 5, wherein the weight ratio of the C8 triglyceride to the C10 triglyceride is from about 55:45 to about 65:35.

7. A method of stabilizing synthetic cannabidiol (CBD) in an oral composition containing same, the method comprising mixing synthetic CBD having a purity of at least 99.8 % with a lipophilic carrier and alpha tocopherol, the carrier consisting of BCP and a mixture of C8 and C10 triglycerides, wherein,
a. the volume ratio of the BCP to the mixture of C8 and C10 triglycerides is from about 1:1 to about 1:3; and
b. the alpha tocopherol is present in an amount from about 0.5 % w/w to about 1.5 % w/w.

8. The method of claim 7, wherein the volume ratio of BCP to the mixture of C8 and C10 triglycerides is about 1:2.

9. The method claim 8, wherein the weight ratio of said C8 triglyceride to said C10 triglyceride is from about 55:45 to about 65:35.

## Patentansprüche

1. Eine stabile Cannabidiol-Zusammensetzung zur Verwendung bei der oralen Verabreichung, die Folgendes umfasst:
a. synthetisches Cannabidiol (CBD) mit einer Reinheit von mindestens 99,5 %, bezogen auf das Gewicht des CBD, in einer Konzentration von etwa 1 % w/w bis etwa 35 % w/w;
b. einen lipophilen Träger in einer Konzentration von etwa 64 Gew.-% bis etwa 98 Gew.-%, wobei der Träger aus Beta-Caryophyllen (BCP) und einem Gemisch aus C8- und C10-Triglyceriden besteht, wobei das Volumenverhältnis von BCP zu dem Gemisch von C8- und C10-Triglyceriden von etwa 1:1 bis etwa 1:3 liegt;
c. Alpha-Tocopherol (Vitamin E) in einer Konzentration von etwa 0,5 Gew.-% bis etwa 1,5 Gew.-% und
d. (fakultativ) von 0 Gew.-% auf eine wirksame Menge von mindestens einem pharmazeutisch verträglichen Hilfsstoff;
wobei die Zusammensetzung im Wesentlichen frei von Verbindungen ist, die aus der Gruppe bestehend aus 3-Caren, Caren, Humulen, α-Pinen, β-Pinen, Linalool, Limonen, γ-Terpinen, Terpinen, Terpineol, Borneol, Eukalyptol, Pulegon, Sabinen, Ocimen, Camphen, Bisabolol, α-Bisabolol, Caryophyllenoxid, Terpinolen, Phytol, Nerolidol, Myrcen, Isophytol, Citronellol, Fenchol, Geraniol, Guaiol, Isopulegol, Menthol, p-Cymen, Phyllandren, Valenzen, Tetrahydrocannabinol (THC), Cannabidolsäure (CBDA), Cannabigerolsäure (CBGA), Cannabinol (CBN), Cannabinolsäure (CBNA), Cannabigerol (CBG), Cannabichromen (CBC), Cannabichromensäure (CBCA), Cannabicyclol (CBL), Cannabicyclolsäure (CBLA), Cannabivarin (CBV), Tetrahydrocannabivarin (THCV), Cannabidivarin (CBDV), Cannabichromevarin (CBCV), Cannabigerovarin (CBGV), Nabilon, Dronabinol, Tetrahydrocannabinolsäure (THCA), Cannabigerolmonomethylether (CBGM), Cannabielsoin (CBE), Cannabicitran (CBT) und Cannabidiol-Dimethylheptyl (CBD-DMH), N-[8-(2-hydroxybenzoyl)amino]caprylat (SNAC)], zusätzliche Lösungsmittel und zusätzliche pharmazeutische Wirkstoffe.

2. Zusammensetzung nach Anspruch 1, wobei das Volumenverhältnis von BCP zu dem Gemisch von C8- und C10-Triglyceriden etwa 1:2 beträgt.

3. Zusammensetzung nach Anspruch 1 oder 2, wobei das CBD in einer Menge von etwa 2 Gew.-% bis etwa 25 Gew.-% vorliegt.

4. Zusammensetzung nach Anspruch 3, wobei das CBD in einer Menge von etwa 9 Gew.-% bis etwa 23 Gew.-% vorliegt.

5. Zusammensetzung eines beliebigen der Ansprüche 1 bis 4, wobei das CBD zu mindestens 99,8 % rein ist.

6. Zusammensetzung eines beliebigen der Ansprüche 1 bis 5, wobei das Gewichtsverhältnis des C8-Triglycerids zum C10-Triglycerid von etwa 55:45 bis etwa 65:35 liegt.

7. Verfahren zum Stabilisieren von synthetischem Cannabidiol (CBD) in einer oralen Zusammensetzung, die dasselbe enthält, wobei das Verfahren das Mischen von synthetischem CBD mit einer Reinheit von mindestens 99,8 % mit einem lipophilen Träger und alpha-Tocopherol umfasst, wobei der Träger aus BCP und einer Mischung aus C8- und C10-Triglyceriden besteht, wobei
a. das Volumenverhältnis des BCP zum Gemisch von C8- und C10-Triglyceriden liegt zwischen etwa 1:1 und etwa 1:3; und
b. das Alpha-Tocopherol liegt in einer Menge von etwa 0,5 Gew.-% bis etwa 1,5 Gew.-% vor.

8. Verfahren nach Anspruch 7, wobei das Volumenverhältnis von BCP zu dem Gemisch von C8- und C10-Triglyceriden etwa 1:2 beträgt.

9. Verfahren nach Anspruch 8, wobei das Gewichtsverhältnis des C8-Triglycerids zu dem C10-Triglycerid von etwa 55:45 bis etwa 65:35 liegt.

## Revendications

1. Composition de cannabidiol stable destinée à être utilisée par voie orale, la composition comprenant :
a. du cannabidiol synthétique (CBD) ayant une pureté d'au moins 99,5 % par rapport au poids du CBD, en une concentration d'environ 1 % p/p à environ 35 % p/p ;
b. un vecteur lipophile à une concentration d'environ 64 % p/p à environ 98 % p/p, dans laquelle le vecteur est constitué de bêta-caryophyllène (BCP) et d'un mélange de triglycérides C8 et C10, dans laquelle le rapport volumétrique de BCP par rapport au mélange de triglycérides C8 et C10 est d'environ 1:1 à environ 1:3 ;
c. de l'alpha-tocophérol (vitamine E), en une concentration d'environ 0,5 % p/p à environ 1,5 % p/p ; et
d. (facultativement) de 0 % p/p à une quantité efficace d'au moins un excipient acceptable sur le plan pharmaceutique ;
dans laquelle la composition est sensiblement exempte de composés choisis dans le groupe consistant en 3-carène, carène, humulène, α-pinène, β-pinène, linalol, limonène, γ-terpinène, terpinène, terpinéol, bornéol, eucalyptol, pulégone, sabinène, ocimène, camphène, bisabolol, α-bisabolol, oxyde de caryophylène, terpinolène, phytol, nerolidol, myrcène, isophytol, citronellol, fenchol, géraniol, guaiol, isopulégol, menthol, p-cymène, phyllandrène, valencène, tétrahydrocannabinol (THC), acide cannabidolique (CBDA), acide cannabigérolique (CBGA), cannabinol (CBN), acide cannabinolique (CBNA), cannabigérol (CBG), cannabichromène (CBC), acide cannabichroménique (CBCA), cannabicyclol (CBL), acide cannabicyclolique (CBLA), cannabivarine (CBV), tétrahydrocannabivarine (THCV), cannabidivarine (CBDV), cannabichromévarine (CBCV), cannabigérovarine (CBGV), nabilone, dronabinol, acide tétrahydrocannabinolique (THCA), éther monométhylique de cannabigérol (CBGM), cannabiélsoïne (CBE), cannabicitrane (CBT), et cannabidiol-diméthylheptyle (CBD-DMH), caprylate de N-[8-(2-hydroxybenzoyl)amino] (SNAC)], des solvants supplémentaires et des agents pharmaceutiquement actifs supplémentaires.

2. Composition selon la revendication 1, dans laquelle le rapport volumique de BCP par rapport au mélange de triglycérides C8 et C10 est d'environ 1:2.

3. Composition selon la revendication 1 ou 2, dans laquelle le CBD est présent en quantité d'environ 2 % p/p à environ 25 % p/p.

4. Composition selon la revendication 3, dans laquelle le CBD est présent en une quantité d'environ 9 % p/p à environ 23 % p/p.

5. Composition selon quelconque des revendications 1 à 4, dans laquelle le CBD est pur à au moins 99,8 %.

6. Composition selon quelconque des revendications 1 à 5, dans laquelle le rapport en poids du triglycéride C8 par rapport au triglycéride C10 est d'environ 55:45 à environ 65:35.

7. Procédé de stabilisation du cannabidiol synthétique (CBD) dans une composition orale le contenant, le procédé comprenant le mélange de CBD synthétique ayant une pureté d'au moins 99,8 % avec un vecteur lipophile et de l'alpha-tocophérol, le vecteur étant constitué de BCP et d'un mélange de triglycérides C8 et C10, dans lequel,
a. le rapport volumique du BCP par rapport au mélange de triglycérides C8 et C10 est d'environ 1:1 à environ 1:3 ; et
b. l'alpha-tocophérol est présent en une quantité d'environ 0,5 % p/p à environ 1,5 % p/p.

8. Procédé selon la revendication 7, dans lequel le rapport volumique de BCP par rapport au mélange de triglycérides C8 et C10 est d'environ 1:2.

9. Procédé selon la revendication 8, dans lequel le rapport en poids dudit triglycéride C8 par rapport audit triglycéride C10 est d'environ 55:45 à environ 65:35.
